# EUROPÄISCHE PATENTSCHRIFT

(11) **EP 0 575 846 B1**
(45) Veröffentlichungstag und Bekanntmachung des Hinweises auf die Patenterteilung: **04.10.2000**
(21) Anmeldenummer: 93109449.4
(22) Anmeldetag: 14.06.1993
(51) Int. Cl.: A23K 1/00, A23L 1/307, A61K 31/33, A23K 1/18, A23K 1/16, A61K 31/365

(54) **Lipasehemmer enthaltende Nahrungsmittel und Futtermittel**
Food and feedstuff containing lipase inhibitors
Produits alimentaires et nourritures pour animaux contenant des inhibiteurs de lipase

(30) Priorität: 24.06.1992 CH 199092
(43) Veröffentlichungstag der Anmeldung: 29.12.1993
(73) Patentinhaber: F. HOFFMANN-LA ROCHE AG, 4002 Basel (CH)
(72) Erfinder: Isler, Dorothea, CH-4056 Basel (CH); Rehm, Walter, CH-4125 Riehen (CH); Widmer, Erich, CH-4124 Münchenstein (CH)
(74) Vertreter: Witte, Hubert, Dr.

(56) Entgegenhaltungen:
- EP-A- 0 129 748
- EP-A- 0 444 482
- WO-A-90/12579
- DE-A- 1 904 239
- DE-A- 4 040 874
- JOURNAL OF NUTRITION, Bd.122, Nr.2, 1992 Seiten 269 - 277 J.A. HENDRICK ET AL 'VARIOUS DIETARY FIBERS HAVE DIFFERENT EFFECTS ON LIPASE-CATALYZED HYDROLYSIS OF TRIBUTYRIN IN VITRO'

## Beschreibung

Die Erfindung betrifft die Verwendung der bei der fermentativen Herstellung von Lipasehemmern, insbesondere Lipstatin, entstehenden Biomassen bei der Herstellung von Nahrungsmitteln, Futtermitteln, Nahrungsmittelzusätzen, Futtermittelzusätzen, Ergänzungsfutter, Fütterungsarzneimittelvormischungen oder oral zu verabfolgenden Arzneimitteln. Ferner betrifft die Erfindung Nahrungsmittel, Futtermittel, Nahrungsmittelzusätze, Futtermittelzusätze, Ergänzungsfutter, Fütterungsarzneimittelvormischungen oder oral zu verabfolgende Arzneimittel enthaltend eine bei der fermentativen Herstellung von Lipasehemmern, insbesondere Lipstatin, entstehende Biomasse und zugesetzte in Wasser unlösliche Rohfasern, wobei das Verhältnis Lipasehemmer zu Rohfasern 0,1 - 100 Gewichtsteile Lipasehemmer zu 2000 bis 3000 Gewichtsteilen Rohfasern beträgt. Die Erfindung betrifft weiter Nahrungsmittel, Futtermittel, Nahrungsmittelzusätze, Futtermittelzusätze, Ergänzungsfutter, Fütterungsarzneimittelvormischungen oder oral zu verabfolgende Arzneimittel enthaltend einen oder mehrere Lipasehemmer, insbesondere Lipstatin oder Tetrahydrolipstatin, und zugesetzte in Wasser unlösliche Rohfasern, wobei a) der/die Lipasehemmer in reiner Form und/oder in Form der bei der fermentativen Herstellung des/der Lipasehemmer(s), insbesondere des Lipstatins, entstehenden Biomasse(n) vorliegt (vorliegen) und b) das Verhältnis Lipasehemmer zu Rohfasern 0,1 - 100 Gewichtsteile Lipasehemmer zu 2000 bis 3000 Gewichtsteilen Rohfasern beträgt.

Tiere haben gewisse Geschmackspräferenzen und ziehen bestimmte Nahrung einer anderen vor. Nur wenn man ein Tier über den Hunger auf eine bestimmte Nahrung dressiert, wird es, wenn der Hunger gross genug ist, diese Nahrung zu sich nehmen. Wenn es aber die Haltungsbedingungen zulassen, wird das Tier, insbesondere der Hund und die Katze, versuchen, ein ihm zusagendes Futter zu fressen, falls dieses im Überfluss vorhanden ist, und dann übergewichtig werden. Dies trifft natürlich zunehmend in den Industrieländern zu.

Ein wesentlicher Faktor für die Akzeptanz eines Futters, vor allem bei Fleischfressern, ist ein bestimmter Fettgehalt im Futter. Eine Fettreduktion im Futter führt erfahrungsgemäss, wenigstens bei Fleischfressern, zu Akzeptanzproblemen. Deshalb ist es z. B. mit Schwierigkeiten verbunden, Hunde auf ein sog. Diätfutter mit reduziertem Fettgehalt umzustellen. Tiere benötigen zur Sättigung ausserdem ein adäquates Nahrungsvolumen.

Die ideale Nahrung für Hunde bzw. Katzen besteht aus einer solchen Mischung von Eiweiss, Kohlehydraten und Fett, sowie Mineralstoffen, Spurenelementen und Vitaminen, die das Idealgewicht der Tiere erhält. Da Tiere dazu neigen, wenn es ihnen gut schmeckt, über den natürlichen Bedarf hinaus Nahrung aufzunehmen, sind etwa ein Drittel aller Hunde und Katzen heute adipös. Der Adipositas könnte vorgebeugt werden, wenn der Hund oder die Katze ein Futter erhält, das folgende Eigenschaften aufweist:
es muss akzeptiert werden, d.h. dem Tier schmecken,
es muss das nötige Volumen aufweisen und sättigen,
die Resorption essentieller Nahrungsbestandteile soll gewährleistet sein,
es darf keine übermässige Gewichtszunahme, die zu einer Adipositas führt, verursachen,
der Kot sollte geformt sein, so dass er leicht eingesammelt werden kann.

Zur Resorption der Nahrungsfette werden die primären Esterbindungen von Triglyceriden durch die Pankreaslipase in freie Fettsäuren sowie Di- und Monoglyceride gespalten. Diese Stoffe werden dann vom Organismus resorbiert und verwertet. Die Lipasehemmer verhindern zu einem wesentlichen Teil die Spaltung der Nahrungsfette und damit die Resorption und Verwertung der Nahrungsfette. Triglyceride werden unverändert ausgeschieden.

Lipasehemmer, wie Lipstatin (LST) und Analoga davon, wie Tetrahydrolipstatin (THL) und N-Formyl-L-leucin-(S)-1-[[(2S,3S)-3-äthyl-4-oxo-2-oxetanyl]methyl]octadecylester (LOC), die im Rahmen der vorliegenden Erfindung verwendet werden, sind in den EPA 129 748, EPA 185 359 und EPA 444 482 beschrieben. Weitere Lipasehemmer sind Esterastin und dessen Derivate wie sie in US 4 189 438 und US 4 202 824 beschrieben sind. Beispiele von bei der fermentativen Herstellung von Lipasehemmern, wie Lipstatin oder Esterastin, entstehenden Biomassen oder Gärkuchen sind in der EPA 129 748 und dem US 4 189 438 beschrieben.

Sinnvollerweise erfolgt der Zusatz der Lipasehemmer zum Futter in Abhängigkeit vom Fettgehalt des Futters. Durch Zusatz von 0,1 bis 100 mg Lipasehemmer pro g Fett, vorzugsweise von 1 bis 50 mg Lipasehemmer pro g Fett im Futter, ist die Herstellung eines idealen Futters für Hunde und Katzen möglich.

Die lipasehemmenden Substanzen können als Futtermittelzusätze, wie sie z. B. in den Futtermittelrichtlinien der EG aufgeführt werden, zum täglichen Futter, insbesondere zum täglichen Alleinfutter gegeben werden. Ein solches Futter würde dann ein konventionelles Diätfutter zur Vorbeugung oder Therapie einer Adipositas überflüssig machen. Sie können auch als Zusatz zu einem Diätfuttermittel, das z.B. eine salzarme Diät enthält, gegeben werden. Es ist auch der Einsatz in einem speziellen Diätfutter denkbar, das zur Vorbeugung oder Therapie einer Adipositas eingesetzt wird.

Die lipasehemmenden Substanzen können auch als Ergänzungsfutter oder als oral zu verabfolgende Arznei durch Gabe in Form von Tabletten, Kapseln, Granulaten, Pulvern, Trinkwasserzusätzen als Einzelzubereitung oder in Kombination oder Mischungen mit andern Bestandteilen von Ergänzungsfuttern oder oral zu verabreichende Arzneien angewendet werden.

Um Lipasehemmer in Form von Fütterungsarzneimitteln, wie sie z. B. in den EG-Richtlinien definiert sind, anzuwenden, können die Lipasehemmer auch Fütterungsarzneimittelvormischungen zugegeben werden.

Durch Zusatz von in Wasser unlöslichen Rohfasern wird die Wirkung der Lipashemmer verbessert. Beispiele solcher Rohfasern sind mikrokristalline Zellulose, z. B. AVICEL, Weizenkleie und Haferkleie. Der Rohfasergehalt im Futter sollte nach Zusatz der Rohfasern zum Fettgehalt des Futters in einem Gewichtsverhältnis von minimal 1/1,5 stehen. Eine Steigerung des Rohfasergehalts ist durchaus möglich, um eine weitere Wirkungssteigerung des Lipasehemmers zu erreichen. Ein optimaler Zusatz von Rohfasern hat ein Gewichtsverhältnis zum Nahrungsfett von 1:2 oder 1:3, d.h. der Rohfasergehalt sollte etwa das Doppelte bis Dreifache des Fettgehalts des Futters betragen. Durch den Zusatz von Rohfasern wird ausser der gesteigerten Verhinderung der Fettresorption auch die Konsistenz des Kots der Tiere erhalten.

Für alle obigen Zwecke kann chemisch hergestellte reine lipasehemmende Substanz oder mikrobiologisch hergestelltes Material, wie es z. B. beim Lipstatin der Fall ist, verwendet werden.

Eine für die Anwendung bei Tieren besonders vorteilhafte Zubereitung ist die Verwendung des Gärkuchens (der Biomasse) in reiner, halbfester oder getrockneter Form. Der Gärkuchen enthält neben Lipstatin auch noch wertvolles Eiweiss und Fett. Von seiner Zusammensetzung her kann der Gärkuchen auch als natürliches, einen Lipasehemmer enthaltendes Nahrungsmittel aufgefasst werden. Durch direkte Zugabe des Gärkuchens zum Futter können andere sonst zugegebene Nahrungsbestandteile eingespart werden, z. B. Eiweiss in Form von Sojamehl und tierische oder pflanzliche Fette.

Die reine Substanz oder den Gärkuchen kann man wie folgt verwenden:
a) als Futterzusatz zum täglichen Futter oder zu Diätfuttern von Tieren insb. von Hunden und Katzen, um das Idealgewicht der Tiere zu erhalten oder zur Vorbeuge und Behandlung der Adipositas bei diesen Tieren,
b) als Ergänzungsfutter oder oral zu verabfolgende Arznei oder als Zusatz zu Ergänzungsfuttern oder oral zu verabfolgende Arzneien, in Kombination mit andern Bestandteilen der Ergänzungsfutter oder Arzneien,
c) als Zusatz zu Fütterungsarzneimitteln oder Fütterungsarzneimittelvormischungen.

Durch diese Verwendung wird folgendes erzielt:
a) eine wesentliche Verhinderung der Fettresorption im Magen-Darmtrakt.
   Die Fettresorption beträgt dabei je nach Dosis des Lipasehemmers und der zugesetzten Rohfasern zwischen 1 und 100% der mit der Nahrung aufgenommenen Fettmenge, vorzugsweise aber etwa 40 bis 70% des Nahrungsfettes.
b) Vorbeuge und Behandlung der Adipositas vornehmlich bei Hunden und Katzen, wobei die Möglichkeit erhalten bleibt, dass die Tiere gesättigt werden und nicht, um den Resthunger zu stillen, auf andere Nahrungsmittel ausweichen,
c) Vorbeuge und Behandlung der Adipositas durch Verfütterung eines Futters oder eines Diätfutters, das die lipasehemmenden Substanzen enthält und das das Tier gerne frisst, d.h. dass die volle Akzeptanz erhalten bleibt, sodass ein mehr oder weniger zwangsweises Dressieren an ein anderes Futter überflüssig wird.
d) Einsparung von Futtermittelbestandteilen pflanzlicher und/oder tierischer Herkunft.
Obiges gilt mutatis mutandis auch für den Menschen.

Ferner ist zu erwähnen, dass Stoffe, die die Resorption des Nahrungsfettes bei Tieren hemmen, zur Bildung von fettarmer Muskulatur der Tiere, insb. der Masttiere eingesetzt werden können.

### Beispiel 1

Nachweis der Wirkung eines Lipasehemmers am Beispiel von Tetrahydrolypstatin (THL) auf die Fettresorption aus der Nahrung und Einfluss von Zellulose auf die THL-Wirkung bei Hunden.

Weibliche Hunde im Alter von 7 bis 15 Jahren und einem Körpergewicht zwischen 13 und 22 kg werden in 2 Gruppen zu je 6 Tieren eingeteilt. Die Tiere werden mit einem Standardfutter für Hunde ernährt. Zum Trinkwasser haben die Tiere freien Zugang.

Am Versuchstag erhalten die Hunde eine Testmahlzeit. Diese enthält als Futterkomponenten 7,5% Protein, 19% Kohlehydrate, 6% Fett, 1,6% Mineralien, 45% Wasser und 20% Ballaststoffe. Das Nahrungsfett besteht zu 58% aus Olivenöl, das mit C-14 Triolein versetzt wurde. Die restlichen Fette setzen sich vorwiegend aus gesättigten Fettsäuren zusammen. Der Lipasehemmer THL und gegebenenfalls die Zellulose werden der Testmahlzeit in den in den Tabellen angegebenen Dosen beigemischt.

Die Resorption des oral mit der Testmahlzeit verabfolgten Fettes wurde an Hand der wiedergefundenen Radioaktivität in den Faeces der Hunde gemessen. Die Differenz zwischen der oral verabfolgten Menge an C-14 markiertem Triolein und der in den Faeces innerhalb 3 Tagen nach Einnahme der Testmahlzeit wiedergefundenen Radioaktivität wird als resorbiertes Fett angesehen. Die resorbierte Menge wird in Prozent der applizierten Menge angegeben. Je weniger resorbiert ist, d.h. je niedriger der Prozentwert ist, je grösser ist die Aktivität des Lipasehemmers.

In Tabelle 1 sind die Ergebnisse aufgezeichnet.

**Tab. 1**

| Wirkung von THL alleine und THL + Zellulose auf die Fettresorption bei Hunden. | | | | | |
|---|---|---|---|---|---|
| | Kontrolle | Gruppe 1 | | Gruppe 2 | |
| Testmahlzeit in g | 420 | 340 | 420 | 340 | 420 |
| Totale Fettmenge in g / Testmahlzeit | 26 | 26 | 26 | 26 | 26 |
| Zellulose in g / Testmahlzeit | 80 | 0 | 80 | 0 | 80 |

| THL-Dosis | | | | | |
|---|---|---|---|---|---|
| in mg / Testmahlzeit | 0 | 20 | 20 | 50 | 50 |
| in mg/kg Kpgw. | 0 | 1,5 | 1,5 | 4 | 4 |
| Trioleinresorption in % der appl. Dosis | 100 | 70 | 48 | 63 | 28 |

In Tabelle 2 wurden die Blutplasmakonzentrationen an totalem Glycerin von Hunden, deren Mahlzeit 50 mg THL + 80 g Zellulose enthielt, mit denen der Kontrolle ohne THL, aber mit Zellulose verglichen.

**Tab.2**

| Vergleich der Blutplasmakonzentrationen nach einer Testmahlzeit mit 50 mg THL und 80 g Zellulose und einer Testmahlzeit mit 80 g Zellulose ohne THL. | | |
|---|---|---|
| Zeit der Messung | Total Plasma-Glycerin (mmol/l ± SEM) | |
| | Kontrolle (80 g Zellulose) | Lipasehemmer (50 mg THL + 80 g Zellulose) |
| Vor Applikation (Ausgangswert) | 0,6 ± 0,05 | 0,7 ± 0,07 |
| 3 Std. nach Applikation | 1,3 ± 0,17 | 0,5 ± 0,02 |
| 7 Std. nach Applikation | 0,8 ± 0,07 | 0,9 ± 0,13 |

### Diskussion der Ergebnisse:

Aus der Tabelle 1 ist ersichtlich, dass der Lipasehemmer THL schon als alleiniger Zusatz zur Testmahlzeit zu einer Reduktion der Resorption des in der Nahrung enthaltenen Fettes, je nach Dosis, um 30 bis 37% führt. Die Aktivität des Lipasehemmers wird erheblich gesteigert, wenn der Testmahlzeit zusammen mit dem Lipasehemmer Zellulose zugefügt wird. In dem Beispiel war nach Zusatz von Zellulose eine Steigerung von 30-37% auf 52-70% der Hemmung der Resorption des applizierten radioaktiv markierten Nahrungsfettes möglich.

Die in Tabelle 1 aufgezeichneten Ergebnisse werden bestätigt durch die Messung der Plasmaglycerinspiegel an Hunden, die eine Testmahlzeit mit 80 g Zellulose-Zusatz mit oder ohne 50 mg THL erhielten. 3 Stunden nach der Einnahme der Testmahlzeit waren die Glycerinkonzentrationen im Plasma der Hunde ohne THL erheblich angestiegen, was auf eine Resorption des applizierten Nahrungsfettes schliessen lässt, während in der Gruppe mit THL es zu keiner Zunahme kam, was die Hemmung der Resorption von Nahrungsfetten bestätigt. Der schwache Anstieg der Resorption 7 Stunden postprandial muss im Zusammenhang mit der attenuierten Fettresorption von 28% gesehen werden.

Der Kot der Hunde war bei den Tieren, die Zellulose allein oder in Kombination mit THL erhielten, fest und geformt, während er bei den Tieren, die THL ohne Zellulose erhielten, eher breiig war.

### Beispiel 2

Nachweis der Wirkung eines Lipasehemmers am Beispiel von Lipstatin (LST, chemisch rein oder als Bestandteil eines Fermentationskuchens) auf die Fettresorption aus der Nahrung bei Mäusen und Hunden und Einfluss von Zellulose auf die Wirkung.

Das Beispiel gliedert sich in 2 Teile. In Teil a) wird ein Versuch mit Mäusen beschrieben, dessen Ergebnisse sich durch einen Versuch mit Hunden (Teil b) bestätigen liessen. In diesem Teil des Versuches wird auch der Einfluss der Zellulose auf die Wirkung des Lipasehemmers untersucht.

In beiden Teilen des Versuches wird als Lipasehemmer mikrobiologisch hergestelltes Lipstatin (LST) eingesetzt. LST wird als chemisch reine Substanz mit LST als Bestandteil eines nach Beispiel 4 von EP 129 748 hergestellten Fermentationskuchens verglichen. Der Gehalt des Kuchens an LST wurde vor Versuchsbeginn mit den üblichen analytischen Methoden auf 4 g LST pro kg frischem Fermentationskuchen bestimmt.

Eine Weender-Analyse zur Bestimmung des Nahrungsmittelgehaltes von Tierfuttern ergab für den Fermentationskuchen folgende Werte:
Gehalt anTrockensubstanz des Fermentationskuchens: 192 g/kg
Gehalt der Trockensubstanz pro kg:

| | |
|---|---|
| Rohasche | 31,4 g |
| Rohprotein | 251,6 g |
| Rohfaser | 9,1 g |
| Rohfett | 499,4 g |
| N-freie Extraktstoffe | 208,5 g |

### a) Mäuseversuch. Vergleich von chemisch reinem LST mit LST als Bestandteil eines Fermentationskuchens.

Reines LST bzw. der getrocknete Fermentationskuchen wurde in einer in pharmakologischen Versuchen gebräuchlichen 5%igen Gummi arabicum/5%iger Milchpulver-Zubereitung suspendiert und als einmalige Gabe in einer Menge von 10 ml/kg Maus oral an 24 Stunden vorgefasteten Tiere unmittelbar im Anschluss an eine flüssige Testmahlzeit appliziert. Die Testmahlzeit enthielt 2,5% Stärke, 24% Glukose, 12% Milchpulver und 7,6% mit C-14 Triolein markiertes Olivenöl. Die Menge an reinem LST bzw. Kuchen ist so zu bemessen, dass die in der Tabelle 3 angegebenen Dosen sichergestellt sind. Danach erhielten die Mäuse ein übliches Standardfutter.

Die Berechnung des resorbierten Nahrungsfettes wurde wie in Beispiel 1 beschrieben durchgeführt.

Die Ergebnisse des Versuches sind in Tabelle 3 aufgezeichnet, wobei die LST-Dosis auf mg/kg Körpergewicht berechnet ist. Die Werte sind Mittelwerte berechnet aus Messungen an jeweils 3 Mäusen pro Test.

**Tab.3**

| Wirkung von reinem LST und LST in einem Fermentationskuchen auf die Fettresorption bei 3 Mäusen pro Dosis. | | |
|---|---|---|
| LST-Dosis in mg/kg | Trioleinresorption in % der appl. Dosis | |
| | LST rein | LST im Ferment.-Kuchen |
| 2,7 | nd | 83 |
| 5,0 | 69 | nd |
| 15 | 52 | nd |
| 18,0 | nd | 54 |
| 50,0 | 41 | nd |

| | | |
|---|---|---|
| nd = nicht bestimmt. | | |

### b) Hundeversuch.

Dieser Versuch wurde analog Beispiel 1 durchgeführt. Als Lipasehemmer diente LST in dem in Teil a) dieses Beispiels beschriebenen Fermentationskuchen. Dieser Kuchen wurde der Testmahlzeit für die Hunde mit zusätzlich 80 g Zellulose pro Testmahlzeit, wie in Beispiel 1 beschrieben, zugemischt.

Die Ergebnisse sind in Tabelle 4 aufgezeichnet.

**Tab.4**

| Wirkung von LST als Inhalt eines Fermentationskuchens alleine oder in Kombination mit Zellulose auf die Fettresorption bei Hunden. | | | | |
|---|---|---|---|---|
| LST-Dosis | | Tierzahl | Trioleinresorption in % der oral appl. Menge | |
| mg/kg Kpgw. | mg/Mahlzeit | N | LST-allein | LST + Zellul. |
| 1,9 | 20 | 2 | nd | 94 |
| 4,3 | 50 | 4 | 86 | 68 |
| 13,9 | 150 | 4 | 69 | 38 |
| 41,0 | 460 | 4 | 33 | 14 |
| ID₅₀ in mg/Mahlzeit | | | 240 | 95 |

Die Differenzen sind im t-Test-Paarvergleich mit p < 0,05 stat. gesichert.

Die Ergebnisse aus dem Hundeversuch (b) bestätigen zunächst die im Versuch a) mit Mäusen erzielten Ergebnisse, dass LST auch dann wenn es in einem Fermentationskuchen enthalten ist, als sehr aktiver Lipasehemmer wirkt. Das war überraschend, denn es musste damit gerechnet werden, dass der Hemmer aus den Zellen, in denen er gebildet wird, nicht freigesetzt wird, wie dies z.B. bei anderen Naturprodukten, wie Vitaminen, der Fall ist. Die aufwendige und teure Isolation des LST aus dem Fermentationskuchen kann also eingespart werden, ohne dass mit einem Wirkungsverlust gerechnet werden muss.

Die Potenz des Lipasehemmers wird in Anwesenheit von Zellulose, wie sie schon in Beispiel 1 beschrieben wird, um einen Faktor 3 gesteigert und konnte auch durch die Ergebnisse des Teils b) des vorliegenden Beispiels 2 belegt werden.

### Beispiel 3

Nachweis der Wirkung eines Lipasehemmers am Beispiel des weiter oben definierten Lipstatinderivates LOC auf die Fettresorption aus der Nahrung bei einmaliger und mehrmaliger Anwendung bei Mäusen.

Im Versuch wurden pro Dosis je 3 Albino-Mäuse im Gewicht von 25 g eingesetzt.

Die Mäuse erhielten während mehrerer Tage eine sog. Fettdiät, deren Fettgehalt so eingestellt war, dass die Mäuse 23,4 g Fett pro kg Kpgw. am Tag erhielten. Die Mäuse wurden 8 Tage mit diesem Futter gefüttert. Am Tag 3 bis 6 des Versuches wurde diesem Futter C-14 markiertes Triolein und LOC in einer Dosis von 50 mg/kg Kpgw. zugemischt. Die Faeces der Mäuse wurden in 24-stündigen Intervallen vom Versuchstag 4 bis 8 gesammelt und nach den in Beispiel 1 angegebenen Richtlinien ausgewertet.

Die Kontrolltiere erhielten jeweils die Testmahlzeit ohne den Wirkstoff LOC.

Die Resorption des oral verabfolgten Fettes wurde wie in den Beispielen 1 und 2 gemessen.

Die Ergebnisse des Versuchs sind in der Tabelle 5 aufgezeichnet:

**Tabl.5**

| Wirkung von LOC nach 1 bzw. 4 tägiger Applikation auf die Fettresorption bei Mäusen. | | |
|---|---|---|
| LOC Dosis | Trioleinresorption in % der oral gegebenen Menge (± SDM) | |
| | 1. Tag | 4. Tag |
| Kontrolle (kein LOC) | 100 | 100 |
| 50 mg/kg Kpgw. | 26 ± 4 | 26 ± 3 |

Aus der Tabelle ist ersichtlich, dass auch nach mehrtägiger Medikation die Wirkung eines Lipasehemmers unverändert ist, d.h. die Resorptionshemmung des Nahrungsfettes unvermindert anhält.

### Beispiel 4

Schondiät mit reduziertem Protein und Kochsalzgehalt für Hunde (die Menge entspricht einer Tagesration für einen 10 kg schweren Hund):
- 200 g: Hühnerleber oder Hackfleisch
- 10 g: Butter
- 100 g: Rohreis
- 60 g: Weizenkleie
- 4 g: Dicalciumphosphat
- 2,5 g: Vitaminmischung, wobei Vitamin A und E in ausreichenden Mengen und in unveresterter Form enthalten sein sollen
- 50 bis 100 g: Lipstatinhaltiger Fermentationskuchen (4 g Lipstatin/kg Kuchen)

Das Fleisch wird in der Butter angebraten und mit dem Reis (salzarm gekocht) vermengt. Im Anschluss daran wird der lipstatinhaltige Fermentationskuchen, die Rohfaser, Mineral- und Vitaminmischung zugegeben und entsprechend konserviert, z. B. in einer 500 g Konservendose. Falls nur eine salzarme Diät erwünscht wird, kann die Fleischmenge erhöht und die Reismenge entsprechend erniedrigt werden.

### Beispiel 5

Simple Mixtur unter Verwendung von lipstatinhaltigem Fermentationskuchen als Ergänzungsfutter:
- 800 g: Hundebüchsenfutter (75% Wasser, 8-13% Protein, 4-7% Fett)
- 200 g: Hundewürfelfutter (15-20% Wasser, 19-22% Protein, 5-15% Fett)
- 80 g: Weizenkleie
- 100 g: Lipstatinhaltiger Fermentationskuchen (4 g Lipstatin/kg Kuchen)
Der Lipstatinfermentationskuchen wird mit der Weizenkleie in die Mahlzeit eingearbeitet, so dass ein Brei entsteht.

Die Menge entspricht einer Tagesration für einen 20 kg schweren Hund, die sinnvollerweise in 2 Portionen gegeben wird.

Eine ausreichende Versorgung mit Vitaminen A und E wird sichergestellt durch Gaben adäquater Dosen 3 Stunden vor oder nach der Mahlzeit.

## Patentansprüche

1. Verwendung der bei der fermentativen Herstellung von Lipasehemmern, insbesondere Lipstatin, entstehenden Biomassen bei der Herstellung von Nahrungsmitteln, Futtermitteln, Nahrungemittelzusätzen, Futtermittelzusätzen, Ergänzungsfutter, Fütterungsarzneimittelvormischungen oder oral zu verabfolgenden Arzneimitteln.

2. Nahrungsmittel, Futtermittel, Nahrungsmittelzusätze, Futtermittelzusätze, Ergänzungsfutter, Fütterungsarzneimittelvormischungen oder oral zu verabfolgende Arzneimittel enthaltend eine bei der fermentativen Herstellung von Lipasehemmern, insbesondere Lipstatin, entstehende Biomasse und zugesetzte in Wasser unlösliche Rohfasern, wobei das Verhältnis Lipasehemmer zu Rohfasern 0,1 bis 100 Gewichtsteile Lipasehemmer zu 2000 bis 3000 Gewichtsteilen Rohfasern beträgt.

3. Nahrungsmittel, Futtermittel, Nahrungsmittelzusätze, Futtermittelzusätze, Ergänzungsfutter, Fütterungsarzneimittelvormischungen oder oral zu verabfolgende Arzneimittel enthaltend einen oder mehrere Lipasehemmer, insbesondere Lipstatin oder Tetrahydrolipstatin, und zugesetzte in Wasser unlösliche Rohfasern, wobei a) der/die Lipasehemmer in reiner Form und/oder in Form der bei der fermentativen Herstellung des/der Lipasehemmer(s), insbesondere des Lipstatins, entstehenden Biomasse(n) vorliegt (vorliegen) und b) das Verhältnis Lipasehemmer zu Rohfasern 0,1 bis 100 Gewichtsteile Lipasehemmer zu 2000 bis 3000 Gewichtsteilen Rohfasern beträgt.

## Claims

1. The use of biomasses obtained in the fermentative production of lipase inhibitors, especially lipstatin, for the manufacture of foodstuffs, feedstuffs, food additives, feed additives, feed supplements, medicinal feed premixes or medicaments for oral administration.

2. A foodstuff, feedstuff, food additive, feed additive, feed supplement, medicinal feed premix or medicament for oral administration containing a biomass obtained in the fermentative production of lipase inhibitors, especially lip statin, and added water-insoluble crude fibres, whereby the ratio of lipid inhibitor to crude fibres amounts to 0.1 to 100 parts by weight of lipid inhibitor to 2000 to 3000 parts by weight of crude fibres.

3. A foodstuff, feedstuff, food additive, feed additive, feed supplement, medicinal feed premix or medicament for oral administration containing one or more lipase inhibitors, especially lipstatin or tetrahydrolipstatin, and added water-insoluble crude fibres, whereby a) the lipase inhibitor(s) is/are present in pure form and/or in the form of (a) biomass(es) obtained in the fermentative production of the lipase inhibitor(s), especially lipstatin, and b) the ratio of lipid inhibitor to crude fibres amounts to 0.1 to 100 parts by weight of lipid inhibitor to 2000 to 3000 parts by weight of crude fibres.

## Revendications

1. Utilisation des biomasses obtenues lors de la préparation fermentative des inhibiteurs de la lipase, en particulier de la lipstatine, pour la préparation de produits alimentaires, d'aliments pour animaux, d'additifs pour produits alimentaires, d'additifs alimentaires pour animaux, d'aliments complémentaires, de prémélanges pour aliments médicamenteux ou de médicaments à administrer par voie orale.

2. Produits alimentaires, aliments pour animaux, additifs pour produits alimentaires, additifs alimentaires pour animaux, aliments complémentaires, prémélanges pour aliments médicamenteux ou médicaments à administrer par voie orale contenant une biomasse obtenue lors de la préparation fermentative des inhibiteurs de la lipase, en particulier de la lipstatine et des fibres brutes ajoutées insolubles dans l'eau, le rapport entre les inhibiteurs de la lipase et les fibres brutes étant de 0,1 à 100 parties en poids d'inhibiteurs de la lipase pour 2.000 à 3.000 parties en poids de fibres brutes.

3. Produits alimentaires, aliments pour animaux, additifs pour produits alimentaires, additifs alimentaires pour animaux, aliments complémentaires, prémélanges pour aliments médicamenteux ou médicaments à administrer par voie orale contenant un ou plusieurs inhibiteur(s) de la lipase, en particulier de la lipstatine ou de la tétrahydrolipstatine et des fibres brutes ajoutées insolubles dans l'eau, a) le ou les inhibiteur(s) de la lipase se trouvant sous une forme pure ou sous la forme de la ou des biomasse(s) obtenue(s) lors de la préparation fermentative de l'inhibiteur ou des inhibiteurs de la lipase, en particulier de la lipstatine et b) le rapport entre le ou les inhibiteur(s) de la lipase et les fibres brutes étant de 0,1 à 100 parties en poids de l'inhibiteur ou des inhibiteurs de la lipase pour 2.000 à 3.000 parties en poids de fibres brutes.
